Europäisches Patentamt

(19) **European Patent Office**　　　(11) Numéro de publication: **0 017 274**

Office européen des brevets　　　　　　　　　　　　　**B1**

⑫　　　　　　**FASCICULE DE BREVET EUROPEEN**

(45) Date de publication du fascicule du brevet:　　(51) Int. Cl.³: **C 12 M 1/00, C 12 P 5/02**
08.12.82

(21) Numéro de dépôt: **80200216.2**

(22) Date de dépôt: **07.03.80**

---

(54) Procédé et installation de traitement des fumiers.

---

(30) Priorité: **14.03.79 CH 2405/79**

(43) Date de publication de la demande:
**15.10.80 Bulletin 80/21**

(45) Mention de la délivrance du brevet:
**08.12.82 Bulletin 82/49**

(84) Etats contractants désignés:
**AT BE DE FR GB IT LU NL SE**

(56) Documents cités:
**FR-A-898 669**
**FR-A-2 240 890**
**US-A-1 892 681**
**US-A-3 838 199**
**US-A-3 973 043**
**US-A-3 981 800**

(73) Titulaire: **SOCIETE AGRICOLE ET FONCIERE S.A.F.**
**S.A., 2, Boulevard Royal, Luxembourg-Ville (LU)**

(72) Inventeur: **Arsovic, Hans Milinko, Beutiweg 1,**
**D-7570 Baden-Baden (DE)**

(74) Mandataire: **Meylan, Robert Maurice, c/o Bugnion SA**
**Conseils en Propriété Industrielle 10, Route de Florissant**
**Case Postale 375, CH-1211 Genève 12 - Champel (CH)**

---

## Procédé et installation de traitement des fumiers

L'invention a pour objet un procédé pour le traitement des fumiers, quels qu'ils soient, notamment bovins, ovins, ou porcins, selon lequel on dilue le fumier, introduit le mélange liquide obtenu dans un bioréacteur de manière à former du biogaz, sépare les particules solides de fumier du liquide, et sèche les particules de fumier récupérées dans un séchoir à brûleur alimenté par le biogaz fourni par le bioréacteur, une partie au moins du liquide séparé étant recyclée et utilisée pour diluer le fumier, le tout de manière à obtenir des résidus riches en protéines utilisables comme aliment pour le bétail et des résidus utilisables comme engrais.

Une telle installation est décrite dans le brevet US 3 838 199. Dans cette installation le mélange liquide, contenant au moins 10% de matière solide, doit subir un temps de séjour de 12 jours et demi dans le bioréacteur et la totalité du liquide résiduel est recyclé. Une telle installation ne pourrait toutefois fonctionner qu'avec des fumiers très propres, c'est-à-dire exempts de paille ou autres débrits végétaux et de minéraux, conditions qui ne se recontrent toutefois pas dans la pratique.

D'autre part, la quantité d'eau recyclée est trop élevée et conduit rapidement à un étouffement de l'installation par excès de sels minéraux. Cet étouffement devrait intervenir au bout de 15 jours environ. En outre, le temps de séjour dans le bioréacteur est trop élevé. Ce temps élevé exige non seulement un très grand gazomètre, mais il se forme dans le bioréacteur une couche flottante de bactéries mortes qui bloquent le système. Enfin, le problème important du traitement de l'ammoniac n'est pas abordé.

Un procédé semblable, mais sans recyclage du liquide résiduel, est décrit dans le brevet US 3 973 043. Il est également prévu que le mélange liquide contienne au moins 10%, en poids, de matière solide. Le temps de séjour dans le bioréacteur est d'au moins 10 jours. Il est nécessaire de décharger périodiquement le bioréacteur, ce qui nécessite l'arrêt de l'installation. Il est prévu d'utiliser tel quel, comme aliment pour le bétail, le résidu de la fermentation anaérobique, ce qui constitue un danger car celui-ci n'est pas stérile. Il est également prévu d'utiliser tel quel, comme engrais, les eaux résiduelles. Or ces eaux, risquent d'entraîner la nitrification du sol. En outre leur transport risque souvent d'être interdit car elles ne sont pas stériles.

Un procédé de nitrification/dénitrification des eaux a été proposé dans le brevet FR 898 669. Ce procédé n'est toutefois applicable qu'à des eaux potables à faible teneur en ammoniac. Or, dans une installation telle que celle faisant l'objet de l'invention, on rencontre de très fortes concentrations d'ammoniac dans les eaux résiduelles. Dans l'installation selon l'art antérieur on utilise un nitrificateur rempli de sable qui a l'inconvénient de retenir les corps morts et qui doit être lavé périodiquement.

La présente invention a pour but de proposer un procédé et une installation pour sa mise en oeuvre susceptibles d'être utilisés dans les conditions réelles d'exploitation, d'un rendement élevé, éliminant tout risque de blocage ou d'étouffement en réduisant le temps de séjour dans le bioréacteur et produisant des résidus stériles, notamment une eau résiduelle stérile et dénitrifiée pouvant sans autre être éliminée dans les canalisations publiques.

Le procédé selon l'invention, tel que défini par la revendication 1 permet d'atteindre ce but.

La combinaison d'un déchiquetage fin du fumier, du contrôle du mélange, de la limitation de la concentration à une valeur au plus égale à 4% et du réchauffage du bioréacteur par le condensat a permis de réduire le temps de séjour dans le bioréacteur à qautre jours et même moins selon les cas, et ce séjour de quatre jours permet d'obtenir une production de gaz de 200 cm$^3$/g, ce qui représente un résultat équivalent à 24 jours selon les courbes établies par le professeur W. Baader, Braunschweig, publiées dans »Biogaz in Théorie und Praxis«, KTBL. La production de méthane commence même le premier jour. L'énergie thermique du séchage est utilisée directement et doublement par la vapeur d'eau et son condensat, tout d'abord dans le bioréacteur, puis dans la sous-installation de nitrification-dénitrification, dans laquelle un apport de chaleur paraît nécessaire.

L'invention a également pour objet une installation, telle que définie par la revendication 3, pour la mise en oeuvre du procédé selon l'invention. Cette installation présente avantageusement des bassins d'oxydation équipés de grilles qui en augmentent la surface biologique efficace et permettent de traiter des eaux présentant une très forte teneur en ammoniac.

Les produits obtenus, soit fines particules de fumier sec, filaments et fibres ainsi que boue peuvent être vendus séparément ou mélangés à volonté.

Une forme préférée de mise en oeuvre du procédé sera décrite, à titre d'exemple, en relation avec le dessin annexé.

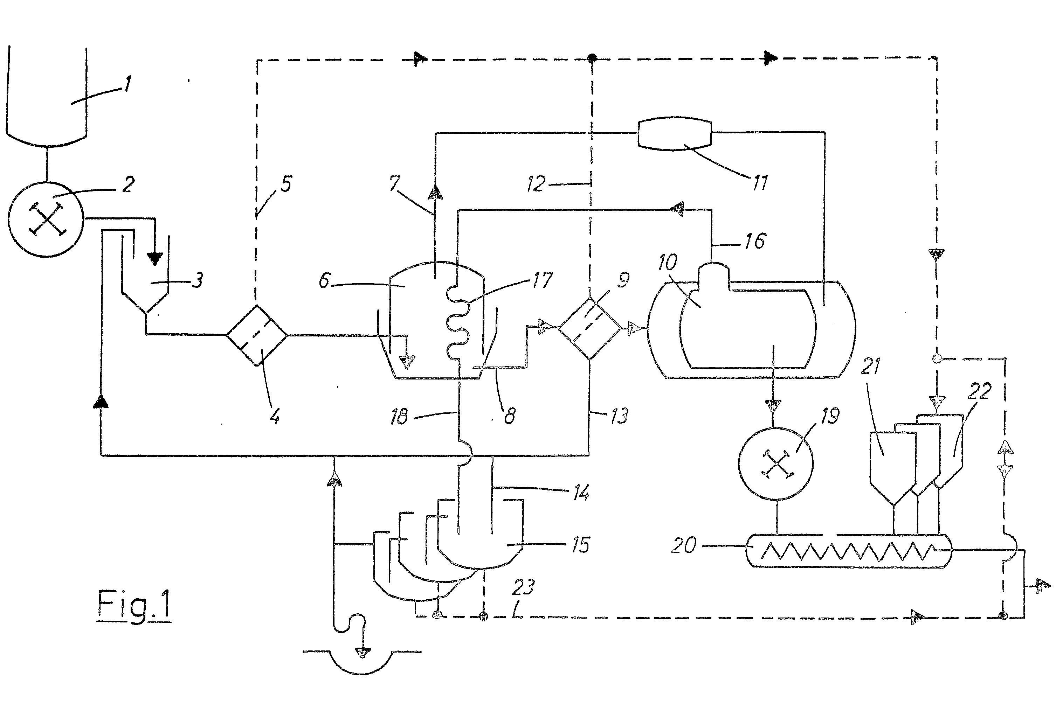

La figure 1 du dessin représente schématiquement l'installation pour la mise en oeuvre du procécé et de diagramme des différents éléments participant à ce procédé.

La figure 2 représente schématiquement l'installation annexe de nitrification et de dénitrification.

Le fumier, provenant directement des exploitations agricoles et des éleveurs est déversé dans un réservoir ou une trémie 1. Il est ensuite finement moulu dans un moulin spécial 2, de préférence, un moulin universel BREMEY 31 fabriqué par Meyer Mühlenbau AG, Soleure (Suisse), qui a pour avantage de ne pas couper mais de déchirer et déchiqueter le matériau, ce qui a pour effet d'augmenter sa surface et d'assurer ainsi une meilleure attaque par les organismes de biodégradation. Le fumier moulu est encuvé dans un bassin mélangeur 3 auquel est ajouté du liquide, par exemple de l'eau, de manière à obtenir un mélange liquide comportant au maximum 4% de matière solide, de préférence 2 à 3%, condition pour une bioréaction favorable dans le bioréacteur. Les particules lourdes, en particulier le sable, sont séparées par décantation pendant l'encuvage dans le réservoir 1.

Le mélange liquide provenant du bassin mélangeur 3 contient une grande quantité de filaments et de fibres végétales provenant de la paille, ainsi que d'autres matières solides légères autres que du fumier. Ces matériaux sont éliminés dans un séparateur 4, par un procédé connu, par exemple par centrifugation, décantage mécanique ou procédé de flottaison. Une vis d'Archimède tournant lentement peut également convenir. Les matériaux solides ainsi éliminés en 5 contiennent environ 20% de protéins. Les éléments filamenteux contiennent à eux seuls 70% d'albumine consommable par le bétail. Le liquide contenant encore de fines particules de fumier ainsi que des filaments et autres particules non séparées, est amené dans un bioréacteur 6, connu en soi, pour la production de biogaz, c'est-à-dire de méthane 7. Dans les installations connues à ce jour on compte avec un temps de séjour de 10 jours dans le bioréacteur pour la production de méthane. Grâce à la présence d'éléments de carbone il a toutefois été possible de réduire considérablement ce temps de séjour pour une même production de gaz.

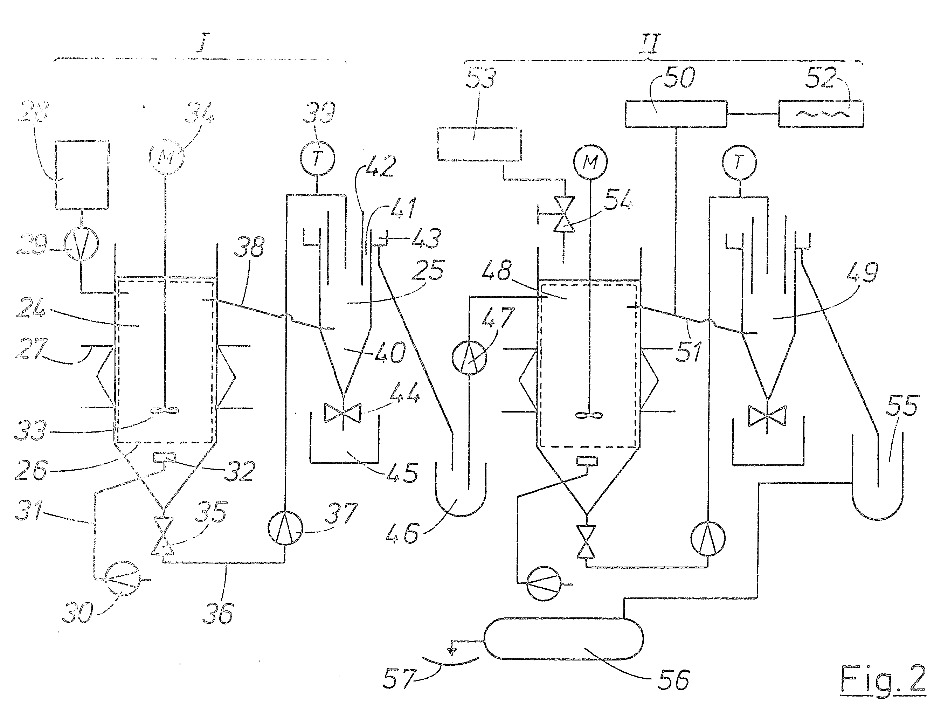

A ce jour une réduction de 60% du temps de passage a pu être atteinte. Le liquide et ses matières en suspension sortant du biore-bioréacteur en 8 est ensuite conduit à un séparateur 9, par exemple un séparateur analogue ou séparateur 4, pour en retirer toutes les substances solides qui sont séchées dans un séchoir 10, de préférence un séchoir à disques de la firme Stord Bartz AS, Bergen (N), comportant un brûleur 11 alimenté par le méthane produit par le bioréacteur 6. Dans le séparateur 9 on recueille encore une certaine quantité de filaments 12 présentant une teneur en protéines d'environ 5%. Ces filaments sont constitués par des fibres non digérées, à cellules très petites, qui flottent facilement. Le liquide séparé 13, qui contient encore des sels et des matières organiques non digérées, est recyclé dans le bassin mélangeur 3 où il constitue l'apport liquide nécessaire pour maintenir la concentration du mélange liquide à une valeur inférieure ou égale à 4%. Le liquide non utilisé 14 est conduit à une installation de nitrification/dénitrification des eaux usées 15. Cette installation sera décrite plus loin, en détail, en relation avec la figure 2.

La vapeur d'eau 16 produite par le séchoir 10 est condensée dans un serpentin 17 monté dans le bioréacteur 6. Cette vapeur d'eau cède donc les calories nécessaires pour que le contenu du réacteur soit amené à une température optimale pour son bon fonctionnement. Le condensat 18, qui contient de l'ammoniac, est déversé dans l'installation de nitrification/dénitrification 15 par la conduite 23.

Le résidu sec fourni par le séchoir 10 est à nouveau finement moulu dans un moulin 19, identique au moulin 2, puis éventuellement mélangé dans un mélangeur 20, par exemple un mélangeur à vis, avec des additifs chimiques 21 ayant pour but d'améliorer la qualité des engrais obtenus. Une partie des fibres et des filaments séparés précédemment peut être également ajoutée en 22 au mélange.

Le procédé de neutralisation de l'ammoniac par nitrification/dénitrification, appliqué au liquide résiduel non recyclé, peut être appliqué au liquide résiduel non recyclé, peut être appliqué d'une manière générale à toutes eaux usées. On a constaté que les concentrations d'ammoniac dépassant 200 mg/l inhibent le processus normal de destruction dans les installations des traitements des eaux usées. D'autre part, dans la technique d'épuration, le nitrite est considéré comme un poison. Il convient donc de neutraliser, par transformation, ammoniac et nitrite. Cette opération s'effectue par dégradation bactérienne ou biosynose, c'est-à-dire par l'action de bactéries. Les bactéries nitrifiantes sont des organismes autotrophes utilisant uniquement des matières minérales pour leur métabolisme. L'espèce nitrosomone dégrade les liaisons ammonium ($NH_4+$) en les transformant en nitrites, tandis que l'espèce nitrobacter oxyde les nitrites en nitrates. Ces réactions biologiques sont optimales pour une température de 30°C et un pH 7,5.

L'installation de nitrification-dénitrification, représentée schématiquement à la figure 2, est constituée essentiellement de deux unités semblables I et II dont le fonctionnement est identique. L'unité I comprend un bassin d'oxydation 24 et un bassin de décantation 25 fonctionnant en circuit fermé. La surface spécifique du bassin d'oxydation 24 est augmentée artificiellement par l'addition de grilles 26 qui créent un support sur lequel bactéries et algues diverses peuvent se développer et augmentent ainsi la surface biologique des bassins. Le bassin peut être chauffé au moyen d'éléments de chauffage 27. Les eaux à traiter sont accumulées dans un réservoir 28 duquel elles sont pompées au moyen d'une pompe 29 dans le bassin d'oxydation 24. L'oxygène nécessaire à la dégradation bactérienne dans le bassin 24 est introduit par une pompe 30, un conduit 31 et un diffuseur 32. De plus, afin d'homogénéiser le milieu il est prévu un agitateur 33 entraîné par un moteur 34. Le fond du bassin d'oxydation 24 est relié à l'entrée du bassin de décantation 25 par une vanne 35, un conduit 36 et une pompe 37 assurant la circulation du liquide. Le retour du bassin de décantation 25 au bassin d'oxydation 24 s'effectue par un conduit 38 par le principe des vases communiquants. Un thermostat 39 placé sur la conduite 36 contrôle les éléments de chauffage 27 de manière à maintenir une température de l'ordre de 27 à 30°C dans le bassin d'oxydation 24, cette température étant idéale pour la nitrification. Le bassin de décantation 25 sert à la séparation de l'eau et des boues formées par les algues et les bactéries qui se sont développées à partir des différents polluants. Le bassin de décantation 25 est de conception connue. Il présente deux zones de repos dans lesquelles la vitesse de circulation des eaux ainsi que les mouvements sont pratiquement nuls, l'une dans la partie inférieure 40 du bassin et l'autre dans la partie supérieure 41, entre la paroi extérieure cylindrique du bassin et une paroi plongeante cylindrique 42. Les boues se déposent dans la zone 40 tandis que c'est dans la zone 41, appelée aussi zone de clarification, que sont recueillies les eaux, séparées de leur boue, par débordement dans un canal annulaire 43. Les boues décantées peuvent être recueillies dans un collecteur 45 à l'aide d'une vanne 44, tandis que les eaux recueillies en 43 s'écoulent librement dans un réservoir intermédiaire 46 dans lequel elles sont pompées par une pompe 47 et introduites dans le bassin d'oxydation 48 de la seconde unité. Le bassin d'oxydation 48 est identique au bassin d'oxydation 24. De même le bassin de décantation 49 est identique au bassin de décantation 25. La seconde unité II ne diffère de la première unité I que par la présence d'un dispositif de mesure du pH 50 branché sur le conduit de retour 51 et associé à un enregistreur 52 enregistrant le pH en continu, et par un réservoir de soude 53 assoicié à une vanne 54 permettant l'addition de soude dans le bassin 48 pour la régularisation du pH. La vanne 53 peut être judiciairement commandée automatiquement par le dispositif de mesure du pH 50.

Les boues formées dans le bassin de décantation 49 sont très légères et une partie de ces boues a tendance à être entraînée par flottation, dans la partie supérieure du bassin. Ces boues sont éliminées dans un décanteur secondaire 55 et les eaux traitées sont recueillies dans un collecteur 56 d'où elles sont évacuées dans un canal 57.

Les conditions de travail et d'utilisation de cette station de nitrification/dénitrification dépendent principalement de la nature des eaux à traiter, en particulier de la teneur en produits organiques et de la teneur en ammoniac.

Si la teneur en produits organiques est importante, la première unité servira en grande partie à la dégradation de ceux-ci. Ce n'est qu'après que les produits organiques sont consommés que commencera la dégradation de l'ammoniac. Selon la teneur en ammoniac on devra alors soit ajouter une troisième unité identique aux deux premières, soit choisir un débit plus faible.

Si la teneur en produit organique est faible, la dégradation de l'ammoniac se fera d'emblée dans la première unité si les bactéries sont adaptées. On pourra alors choisir, selon la concentration en ammoniac le débit à travers la station ou si celle-ci est très importante, ajouter une troisième unité identique aux deux premières.

Les essais ont été effectués en laboratoire avec une station présentant les valeurs suivantes:

| | |
|---|---|
| — contenance du bassin d'oxydation | 12 l |
| — contenance du bassin de décantation | 1,5 l |
| — contenance du bassin de décantation secondaire | 1,5 l |
| — Débit à travers la station pour une concentration d'ammoniac d'environ 1 g/l | 6 à 8 l/24 h |
| — Débit des pompes du circuit fermé | 20 l/h |
| — Débit des pompes d'oxygénation | ~30 l/h |

Cette station de nitrification-dénitrification se distingue des installations connues par la charge très élevée avec laquelle elle peut travailler, par la zone de température dans laquelle elle travaille, par le degré d'élimination de l'influence des carbones et par la transformation de l'azote en présence de telles quantités de soufre. La boue résiduelle se compose de protéines utilisables comme aliment pour le bétail.

Pour l'ensemble du procédé de traitement des fumiers, les chiffres suivants ont pu être mesurés:

Pour un fumier frais contenant 17% de matière solide, c'est-à-dire présentant un taux d'humidité de 83%, il reste, dans le liquide en suspension arrivant dans le bioréacteur 6,7% de substances sèches en suspension. Les substances séchées demeurées dans la solution à 7% peuvent théoriquement être transformées microbiologiquement à 4,83%. Le résultat des analyses indique que 63% de ces substances séchées sont transformées en gaz, le reste soit 37%, restant dans le liquide 8 sortant du bioréacteur. Dans ce liquide on trouve 0,35% de protéines correspondant à une teneur en protéines de 19,8% à l'état sec.

Le bioréacteur est capable de fournir entre 80 et 120% de l'énergie nécessaire au séchage, respectivement à l'évaporation de l'eau en surplus. La condensation de la vapeur d'eau provenant du séchoir dans le bioréacteur et le chauffage de la masse biologique dans le bioréacteur élève la température de 40% à l'entrée du séchoir. Ainsi, le liquide froid qui entre dans le bioréacteur en ressort à une température d'environ 35°C. L'énergie ainsi épargnée est considérable.

Le bilan de la production d'énergie de l'installation est approximativement le suivant: 1000 kg de fumier contenant 17% de matériel sec produit 100 à 120 kg de filaments contenant des protéines qui peuvent directement être centrifugées, décantées ou tamisées.

Dans les suspensions restantes de 70 kg/m³ en solution à 5,3%, on obtient environ 80 kg de matériau sous forme de filaments, cellulose, minéraux et de résidus divers, albumine, ainsi que des impuretés en suspension. 30% de 70 kg/m³, en solution à 5,3%, sont transformés biologiquement en méthane, $CO_2$, acides, protéines, eau et composés d'azote.

Après le passage dans le bioréacteur il reste, en plus du liquide résiduel, environ 150 à 180 kg de substance utilisable, c'est-à-dire commercialisable.

L'augmentation que l'on constate entre 150 kg de substances récupérées par tonnes de fumier et 150 à 180 kg par tonne est conditionnée par la biosynose dans le bioréacteur.

La boue récupérée, en 23, dans l'installation de nitrification/dénitrification des eaux usées 15 contient jusqu'à 90% d'albumine digestible par le bétail. Cette boue peut être vendue comme aliment avec les fibres séparées en 5 et 12 ou mélangée au produit sec fournit par le séchoir dans le but d'obtenir un plus grand volume. Selon la situation du marché on peut ajouter des particules de cellulose ou des filaments précédement éliminés. Le produit sec peut être soit mis en sac, soit mis en tas et vendu.

L eprocédé se distingue donc par une grande flexibilité susceptible d'éliminer les fluctuations de marché.

## Revendications

1. Procédé de traitement des fumiers selon lequel on dilue le fumier, introduit le mélange liquide obtenu dans un bioréacteur de manière à former du biogaz, sépare les particules solides de fumier du liquide, et sèche les particules de fumier récupérées dans un séchoir à brûleur alimenté par le biogaz fourni par le bioréacteur, une partie au moins du liquide séparé étant recyclée et utilisée pour diluer le fumier, le tout de manière à obtenir des résidus riches en protéines utilisables comme aliment pour le bétail et des résidus utilisables comme engrais, caractérisé en ce qu'on déchiquète finement le fumier, qu'on maintient la concentration du mélange fumier-liquide à une valeur au plus égale à 4%, qu'on sépare du mélange liquide les filaments, fibres et autres matériaux solides autres que le fumier avant l'introduction du mélange dans le bioréacteur, qu'on active la vioréaction au moyen de l'énergie thermique de la vapeur d'eau produite par le séchage, qu'on soumet le liquide résiduel du séchage non recyclé à un traitement de nitrification/dénitrification par dégradation bactérienne et oxygénation et par séparation des boues par décantation, en opérant par recirculation du liquide décanté, à température et pH contrôlés en réchauffant le liquide résiduel par l'addition du condensat de la vapeur d'eau de séchage, qu'on recycle, selon les besoins, une partie de l'eau dénitrifiée, stérilisée et déminéralisée, au mélange initial pour en réduire la concentration, et qu'on récupère les boues résiduelles, riches en protéines.

2. Procédé selon la revendication 1, caractérisé en ce qu'on mélange une partie au moins des fibres et filaments précédemment éliminés, avec le produit solide sé⁻ ʰé.

3. Installation pour la mise en oeuvre du procédé selon la revendication 1, caractérisée en ce qu'elle comprend un moulin (2) pour déchiqueter le fumier, un bassin mélangeur (3) alimenté par du liquide, un premier séparateur (4) séparant les filaments et fibres végétales provenant de la paille, un bioréacteur (6), un second séparateur (9) dans lequel est conduit le mélange liquide résiduel sortant du bioréacteur, un séchoir (10) comportant un brûleur (11) alimenté par le méthane produit par le bioréacteur, un serpentin (17) situé dans le bioréacteur et dans lequel est condensée la vapeur d'eau produite par le séchoir, un conduit (13) reliant le second séparateur (9) au bassin mélangeur (3) pour l'alimenter en liquide au moyen du liquide résiduel recyclé, ce conduit présentant un embranchement (14) débouchant dans une sous-installation de nitrification/dénitrification (15) comprenant au moins deux unités en série (I, II) comprenant chacune un bassin d'oxydation (24, 48) et un bassin de décantation (25, 49) reliés entre eux en circuit fermé, chacun des bassins d'oxydation comprenant des moyens d'adduction d'air (31), la première unité (I) traitant les produits organiques et la seconde unité (II) l'ammoniac, ledit serpentin (17) débouchant également dans la sousinstallation de nitrification/dénitrification.

4. Installation selon la revendication 3, caractérisée en ce que les bassins d'oxydation (24, 48) contiennent des grilles (26) destinées à augmenter la surface biologique des bassins.

5. Installation selon la revendication 4, caractérisée en ce que le deuxième bassin d'oxydation (48) est associé à un dispositif automatique de mesure et de correction du pH par addition de soude (50, 52, 53).

## Patentansprüche

1. Verfahren zur Behandlung von Mist, wobei man den Mist verdünnt und die erhaltene flüssige Mischung in einen Bioreaktor zur Bildung von Biogas überführt, dann die festen Bestandteile des Mistes von der Flüssigkeit trennt und die gewonnenen Feststoffe des Mistes in einer Trockenkammer mit einem Brenner trocknet, welcher von dem Biogas aus dem Bioreaktor gespeist wird, und wobei man wenigstens einen Teil der abgetrennten Flüssigkeit zurückleitet und zum Verdünnen des Mistes verwendet zwecks Erlangung von an Proteinen reichen, als Nahrung für Vieh verwendbaren Rückständen sowie von als Dünger verwendbaren Rückständen, dadurch gekennzeichnet, daß man den Mist fein zerkleinert und die Konzentration der Mist/Flüssigkeit-Mischung auf einen Wert von höchstens 4% hält, daß man von der flüssigen Mischung Fäden, Fasern und andere Feststoffe außer den Mist vor dem Einbringen der Mischung in den Bioreaktor abtrennt, daß man die Bioreaktion mittels der thermischen Energie des Wasserdampfes von der Trocknung fördert, daß man die bei der Trocknung zurückbleibende, nicht zurückgeleitete Flüssigkeit einer Nitrier-/Denitrier-Behandlung durch bakteriellen Abbau und Oxydation sowie durch Abtrennung des Schlammes durch Dekantieren unterwirft, indem man durch Rückleitung der dekantierten Flüssigkeit bei gesteuerter Temperatur und gesteuertem pH-Wert arbeitet und die zurückbleibende Flüssigkeit durch Zusatz von Kondensat des Wasserdampfes von der Trocknung wieder aufheizt, daß man je nach Bedarf einen Teil des denitrierten, sterilisierten und entsalzten Wassers in die ursprüngliche Mischung zurückleitet, um deren Konzentration herabzusetzen, und daß man den zurückbleibenden, an Proteinen reichen Schlamm gewinnt.

2. Verfahren nach Anspruch 1, dadurch gekennzeichnet, daß man wenigstens einen Teil der vorher abgetrennten Fasern und Fäden mit den getrockneten Festprodukten mischt.

3. Vorrichtung zur Durchführung des Verfahrens nach Anspruch 1, gekennzeichnet durch eine Mühle (2) zur Zerkleinerung des Mistes, durch ein von der Flüssigkeit gespeistes Mischbecken (3), durch einen ersten Separator (4) zur Abtrennung der pflanzlichen Fäden und Fasern aus dem Stroh, durch einen Bioreaktor (6), durch einen zweiten Separator (9), in welchen die flüssige zurückbleibende Mischung aus dem Bioreaktor geleitet wird, durch eine Trockenkammer (10) mit einem von dem Methan aus dem Bioreaktor gespeisten Brenner (11), durch eine Schlange (17) in dem Bioreaktor, in welcher der Wasserdampf aus der Trockenkammer kondensiert wird, durch eine den zweiten Separator (9) und das Mischbecken (3) zur Flüssigkeitszufuhr von zurückbleibender zurückgeleiteter Flüssigkeit verbindende Leitung (13) mit einer Abzweigung (14), die in eine Unternitrier-/-denitriervorrichtung aus wenigstens zwei in Reihe angeordneten Einheiten (I, II) mündet, von denen jede ein Oxydationsbecken (24, 48) und ein Dekantierbecken (25, 49) aufweist, die in einem geschlossenen Kreislauf miteinander verbunden sind, und wovon jedes der Oxydationsbecken mit Luftzufuhreinrichtungen (31) versehen ist und die erste Einheit (I) die organischen Produkte und die zweite Einheit (II) das Ammoniak behandelt und wobei die Schlange (17) ebenfalls in der Unternitrier-/-denitriervorrichtung mündet.

4. Vorrichtung nach Anspruch 3, dadurch gekennzeichnet, daß die Oxydationsbecken (24, 48) Gitter (26) zur Vergrößerung der biologischen Oberfläche des Beckens enthalten.

5. Vorrichtung nach Anspruch 4, dadurch gekennzeichnet, daß das zweite Oxydationsbecken (48) mit einer automatischen Einrichtung zur Messung und Korrektur des pH-Wertes durch Zusatz von Soda (50, 52, 53) ausgestattet ist.

## Claims

1. Process of treating manures wherein the manure is diluted, the resultant liquid mixture is introduced into a bioreactor to yield biogas, the solid manure particles are separated from the liquid, and the recovered manure particles are dried in a burnertype drier operating with the biogas produced in thebioreactor, at least one portion of the separated liquid being recycled and re-used for diluting the manure, the whole with a view to produce high-protein residues usable as fertilizers, characterized in that the manure is finely shredded, the manure-liquid mixture concentration is kept to a value not in excess of 4%, the filaments, fibres and other solid materials other than manure are separated from the liquid mixture before introducing the mixture into the bioreactor, the bioreaction is activ   d by means of the thermal energy of water vapor produced during the drying step, the residual non-recycled liquid from the drying step is subjected to a nitrification/denitrification treatment by bacterial degradation and oxygenation and by separating the sludge by setteling, by recirculating the settled liquid at temperature and pH values controlled by re-heating the residual liquid through the addition of the condensate from the water vapor resulting from the drying step, and one portion of the denitrified sterilized and demineralized water ist recycled according to requirements in the initial mixture for reducing the concentration thereof, the residual high-protein sludge being eventually regenerated.

2. Process according to claim 1, characterized in that at least one portion of the previously eliminated fibres and filaments is mixed with the dried solid product.

3. Apparatus for carrying out the method of claim 1, characterized in that it comprises a grinder (2) for shredding the manure, a mixing tank (3) fed with liquid, a first separator (4) for separating the vegetable filaments and fibres derived from the straw, a bioreactor (6), a second separator (9) for receiving the liquid residual mixture from the bioreactor, a drier (10) comprising a burner (11) fed with the methane produced by the bioreactor, a coil piping (17) disposed inside the bioreactor and in which the water vapor produced by the drier is condensed, a conduit (13) connecting the second separator (9) to the mixing tank (3) for supplying liquid thereto from the recycled residual liquid, said conduit having a branch section (14) opening into a nitrification/denitrification sub-plant (15) comprising at least two units in series (I, II) comprising each an oxidation tank (24, 48) and an settling tank (25, 49) interconnected in closedcircuit relationship, each oxidation tank comprising air supply means (31), the first unit (I) treating the organic products and the second unit (II) the ammonia, said coil piping (17) also opening into said nitrification/denitrification sub-plant.

4. Apparatus according to claim 3, characterized in that said oxidation tanks (24, 48) comprise grid means (26) adapted to increase the biological surface area of the tanks.

5. Apparatus according to claim 4, characterized in that the second oxidation tank (48) is associated with a device for automatically measuring and correcting the pH by the addition of soda (50, 52, 53).

**Fig.1**

0017274

Fig.2